# EUROPEAN PATENT APPLICATION

(11) **EP 0 834 323 A1**
(43) Date of publication of application: **08.04.1998**
(21) Application number: 96202725.6
(22) Date of filing: 30.09.1996
(51) Int. Cl.: A61K 48/00, A61K 38/19, A61K 38/20, A61K 45/05

(54) **Cytokine gene therapy for treatment of malignacies**

(71) Applicant: Introgene B.V., NL-2288 GJ Rijswijk (NL)
(72) Inventor: The designation of the inventor has not yet been filed
(74) Representative: Smulders, Theodorus A.H.J., Ir.

(57) **Abstract**

The present invention provides novel means and methods for eliminating unwanted cells or organisms from a (mammalian) subject. In particular tumors can be combatted using the compositions according to the present invention. In a preferred embodiment solid tumors are injected with a burst of cytokine (in particular IL-1α or IL-3) together with a slow release source for a cytokine. To further boost the immune system an adjuvans is preferred. In a preferred embodiment the source for the slow release cytokine is a recombinant adenovirus. In an especially preferred embodiment the adjuvans comprises inactivated (cracked) adenovirus producing cells.

## Description

### Introduction

The invention relates to the field of the treatment of mammals suffering from a disease involving the presence of a certain population of harmful cells or organisms . These diseases include various forms of cancers (tumors), autoimmune diseases and infectious diseases caused by bacteria, viruses or other microorganisms. The first two kinds of diseases involve the uncontrolled growth of a certain subset of cells of the mammal to be treated itself. These diseases have been found difficult to treat, particularly because the underlying mechanisms are not clear. The same goes for a certain group of infectious diseases, such as AIDS. AIDS, cancer and autoimmunediseases all have in common that they involve some kind of failure of the immune system. All the diseases mentioned above will together be referred to as malignancies for the sake of simplicity. The present invention gives a novel approach, i.e. methods and means for the treatment of malignancies involving recombinant DNA technology, more in particular involving gene therapy.

Gene therapy is a recently developed concept for which a wide range of applications can be and have been envisaged.

In gene therapy a molecule carrying genetic information is introduced in some or all cells of a host, whereby the genetic information is added to the host in a functional format.

The genetic information added may be a gene or a derivative of a gene, such as a cDNA, which encodes a protein. In this case the functional format means that the protein can be expressed by the machinery of the host cell. Applications include the treatment of genetic disorders by supplementing a protein or other substance which is, through said genetic disorder, not present or at least present in insufficient amounts in the host, the treatment of tumors and (other) acquired diseases such as (auto)immune diseases or infections, etc.

The present invention is in particular directed towards the removal or elimination of unwanted substances (organisms or cells (herein referred to as malignancies)) and towards application of a recombinant vaccine (for the treatment of malignancies).

The invention thus provides a composition for the treatment of malignancies comprising a relatively high burst dose of at least one cytokine or a functional equivalent or fragment thereof, a source for sustained release of a significantly lower but effective dose of at least one cytokine or a functional equivalent or fragment thereof together with a suitable vehicle for administration. In the past years, a new approach to enhance the immune response against solid experimental tumors has been described. The concept underlying these studies is to have cytokines produced at high levels inside the tumor, while systemic concentrations remain low, thereby reducing or preventing the toxic side effects of these proteins. The ultimate goal of these approaches is to obtain regression of the treated tumor and simultanously induce such a high degree of immunity that coexisting metastases are also destroyed. Among the end goals in such studies are:
1. tumor specific immunity following cytokine assisted vaccination.
2. complete or partial regression of directly treated tumors.
3. prolonged survival.
4. prevention of the development of metastases-or regression of established tumors by so-called therapeutic vaccination with transduced tumor cells expressing the cytokine.

One or more of such effects have been obtained with transduced tumor cells expressing IL-2 (Fearon *et al.*, 1990; Gansbacher *et al*., 1990b), IL-4 (Golumbek *et al*., 1991; Platzer *et al.*, 1992), interferon-gamma (Gansbacher *et al.*, 1990a), interferon-alpha (Ferrantini *et al.,* 1993), TNF alpha (Blankenstein *et al.*, 1991)^{,} IL-7(McBride *et al.*, 1992) (Hock *et al*., 1991) G-CSF (Colombo *et al*., 1991) GM-CSF (Dranoff *et al.*, 1993) IL-12 (Tahara *et al.,* 1994) and several other cytokines, among them IL-1 (Apte *et al.,* 1994a; Apte *et al*., 1994b; Douvdevani *et al.,* 1992; Nakata *et al.,* 1988; Zîller *et al.,* 1992) and IL-3 (McBride *et al.*, 1993; Pulaski *et al.,* 1993; Pulaski et al, 1996).

The introduction of cytokine genes was originally conceived as a means to induce costimulatory signals into tumors (or tumor cells) for antigen presentation. The data now available suggest that in many cases the cytokine elicits an inflammatory type cell infiltration that results in improved antigen presentation by mechanisms yet unknown and possibly varying. During an intense local inflammation, invading cells may destroy normal tissues - as seen in the joints in rheumatoid arthritis- as well as tumor cells. These killed cells are lysed and may release tumor antigens in a form that can be presented by other subpopulations of the invaders to T lymphocytes and thus elicit an immune reaction.

However, once these cells are lysed the production of cytokines stops. In the present invention, the same initial burst of cytokine or a functional equivalent thereof as provided by the prior art is achieved, but also a source for sustained release of a cytokine at a lower level but over a prolonged period of time is provided. In this way the inflammatory state can be prolonged significantly thereby allowing more time for immunity against the malignancy to be developed. For the purposes of this invention functional equivalents of cytokines (the higher and the lower doses may be the same or different cytokines) are defined as those molecules derived from the original cytokine having similar activity as the original cytokine (in kind not in amount). Herein it is understood that where cytokine is written the functional equivalents are also meant unless explicitly stated otherwise. The burst release of cytokine may simply be provided by a high dose of the cytokine. The difficult part is providing a sustained release of the lower dose of cytokine at the site or in the vicinity of the malignancy. A very suitable composition according to the invention thus comprises a relatively high burst amount of a cytokine or an equivalent thereof further comprising a source for a lower dose of cytokine or an equivalent thereof, wherein the source of the lower dose of a cytokine is a gene delivery vehicle, which should be capable of transducing the malignancy in a functional manner, i.e. capable of producing the cytokine.
Such a gene delivery vehicle preferably comprises a recombinant vector, more preferably a viral vector, in particular an adenoviral vector. When the source of the lower dose of a cytokine is a recombinant adenovirus this virus should be capable of infecting the malignancy.

Adenoviruses carrying certain deletions have been proposed as suitable vehicles for gene therapy. Gene-transfer vectors derived from adenoviruses (so-called adenoviral vectors) have a number of features that make them particularly useful for gene transfer for such purposes. Eg. the biology of the adenoviruses is characterized in detail, the adenovirus is not associated with severe human pathology, the virus is extremely efficient in introducing its DNA into the host cell, the virus can infect a wide variety of cells and has a broad host-range, the virus can be produced in large quantities with relative ease, and the virus can be rendered replication defective by deletion of the early-region 1 (E1) of the viral genome.

The adenovirus genome is a linear double-stranded DNA molecule of approximately 36000 base pairs with the 55-kDa terminal protein covalently bound to the 5' terminus of each strand. The Ad DNA contains identical Inverted Terminal Repeats ITR of about 100 base pairs with the exact length depending on the serotype. The viral origins of replication are within the ITRs exactly at the genome ends.

During the productive infection cycle, the viral genes are expressed in two phases: the early phase, which is the period upto viral DNA replication, and the late phase, which coincides with the initiation of viral DNA replication.
During the early phase only the early gene products, encoded by regions E1, E2, E3 and E4, are expressed, which carry out a number of functions that prepare the cell for synthesis of viral structural proteins (Berk, 1986). During the late phase the late viral gene products are expressed in addition to the early gene products and host cell DNA and protein synthesis are shut off. Consequently, the cell becomes dedicated to the production of viral DNA and of viral structural proteins (Tooze, 1981).

The E1 region of adenovirus is the first region of adenovirus expressed after infection of the target cell. This region consists of two transcriptional units, the E1A and E1B genes. The main functions of the E1A gene products are i) to induce quiescent cells to enter the cell cycle and resume cellular DNA synthesis, and ii) to transcriptionally activate the E1B gene and the other early regions (E2, E3, E4).

Vectors derived from human adenoviruses, in which at least the E1 region has been deleted and replaced by a gene-of-interest, have been used extensively for gene therapy experiments in the pre-clinical and clinical phase.

As stated before all adenovirus vectors currently used in gene therapy have a deletion in the E1 region, where novel genetic information can be introduced. The E1 deletion renders the recombinant virus replication defective (Stratford-Perricaudet and Perricaudet, 1991). We have demonstrated that recombinant adenoviruses are able to efficiently transfer recombinant genes to the rat liver and airway epithelium of rhesus monkeys (Bout *et al*., 1994a; Bout *et al.*, 1994b). In addition, we (Vincent *et al*., 1996a; Vincent *et al.*, 1996b) and others (for reviews see Blaese et al, 1995 and Smith, 1995) have observed a very efficient *in vivo* adenovirus mediated gene transfer to a variety of tumor cells *in vitro* and to solid tumors in animals models (lung tumors, glioma) and human xenografts in immunodeficient mice (lung) *in vivo*.

In contrast to for instance retroviruses, adenoviruses a) do not integrate into the host cell genome; b) are able to infect non-dividing cells and c) are able to efficiently transfer recombinant genes *in vivo* (Brody *et al.*, 1994). Those features make adenoviruses attractive candidates for *in vivo* gene transfer of, for instance, suicide or cytokine genes into tumor cells.
Preferred cytokines according to the invention are those cytokines giving an inflammation-like response. The preferred cytokines particularly include interleukin-3 or interleukin-1 alpha or functional equivalents or fragments or mixtures thereof.
A detailed description on interleukin 1 has been provided in our patent application EP 95 20 2213. Briefly, IL-1 is one of the most prominent mediators of inflammation. It has a wide range of biological activities related to inflammatory reactions induced by infections and by certain immunological responses. Among others, it has chemotaxic properties, it potentiates activation and proliferation of T lymphocytes and macrophages, it stimulates the maturation of Langerhans cells into immunostimulatory dendritic cells (Heufler *et al*., 1988) and promotes the recovery of the hemopoietic system following irradiation (Blazar *et al*., 1992; Schwartz *et al*., 1988). IL-1 is one of the most potent inducers of cytokine production by a diversity of cells. Its anti-tumor activity has been studied most extensively by the group of Apte (Apte *et al.*, 1994a; Apte *et al.*, 1994b; Douvdevani *et al.*, 1992). These investigators studied several mouse fibrosarcomas which did or did not constitutively express IL-1 a upon stimulation. Expression of IL-1 alpha was also induced in non-expressing tumor cell lines by gene transfer using retroviral vectors. They found a strong correlation between IL-1 expression and reduced tumorigenicity. Also regression of IL-1 expressing tumors induced the development of systemic immunity as manifested by rejection of a challenge with a non IL-1 producing tumor cell line. They also obtained regression of a non expressing tumor by treating the hosts with tumor cells that did express IL-1 (Apte *et al*., 1994b).

Nakata et al (Nakata *et al*., 1988) obtained tumor regression and even cure of smaller tumors following repeated systemic or intra-tumor injection of recombinant hu IL-1α (the protein) in several mouse tumors. The effect was IL-1 dose dependent and the effect was strongest in immunogenic tumors. Cured animals were resistant to subsequent challenge with the same tumor.
However, as stated before, in all these cases an initial burst dose of IL-1 alpha is given, but no sustained release of a cytokine is provided. The effect of sustained release of IL-1 alpha can be very clearly seen from the following.

After *in vivo* administration of living IL-1α adenovirus producer cells (IG.Ad.MLP.hIL-1α-293 cells) into experimental rat tumors, we have observed regression not only of the injected tumor but also of non-treated tumors growing at a distant site. These results show that a host response against the tumor is elicited when high doses of cytokines are produced locally in the tumor.

As stated before IL-3 is another preferred cytokine to be used in the present invention.
Murine interleukin-3 (IL-3) was initially purified from medium conditioned by myelomonocytic leukemia WEHI-3 cells (Ihle *et al.*, 1983). The isolation and cloning of the murine cDNA (Fung *et al.*, 1984; Kindler *et al*., 1986; Yokota *et al*., 1984) revealed a single open reading frame that specifies a polypeptide of 166 amino acids (aa). The encoded protein was shown to be processed at the N-terminal site by removal of 26 residues to yield a mature protein comprising 140 amino acids. The protein sequence of mature processed mIL-3 indicated a molecular weight of 15,102 dalton with four potentially N-linked glycosylation sites and four cysteine residues. Glycosylation is not required for biological activity *in vitro*, but it remains to be elucidated whether glycosylation affects the biological activity *in vivo*. It is hypothesized that the degree or type of glycosylation could regulate interaction with the extracellular matrix and influence diffusion or localization in tissues (Ziltener *et al.*, 1988).

The coding regions for mature mouse and rat IL-3 share 76% sequence homology, the amino acid sequence homology is 54%.

Among human growth factors, IL-3 is the only factor to be predominantly if not exclusively produced *in vitro* by activated normal T-cells (Niemeyer *et al.*, 1989). Studies on IL-3 expression in human T-cell clones revealed that IL-3 expression is restricted to a small subset of T-cells and NK cells and that IL-3 expression can be enhanced by mitogens and antigens, or even more profoundly by IL-2 (Wimperis *et al.,* 1989). Recently, it has been shown that in addition to activated T-cells other cell types such as activated murine mast cells, murine keratinocytes, eosinophils, neutrophils, thymic epithelial cells, neurons and astrocytes are capable of (low level) IL-3 expresssion (Kita *et al*., 1991; Konishi *et al*., 1994).

Interleukin-3 (IL-3) is a cytokine well described as a hematopoietic growth factor that has a wide range of target cells including progenitor cells of every lineage, excluding cells committed to the T and B lymphoid lineage (Schrader *et al*., 1988).

The main production of IL-3 by activated T cells has led to the hypothesis that IL-3 is not involved in steady-state hematopoiesis but functions as a link between the T lymphocytes of the immune system, which senses invasion of the body by foreign materials, and the hematopoietic system which generates the cellular elements that mediate defence and repair responses (Ihle, 1989).

IL-3 exerts a broad spectrum of biological functions (Ihle *et al*., 1983), including stimulatory activity on several myeloid leukemia cell lines, formation of granulocyte-macrophage colonies, mast cell growth factor activity, P cell-stimulating activity and histamine producing cell-stimulating factor activity. In addition, IL-3 is capable to promote the proliferation of megakaryocyte colony-forming cells, to support the differentiation of eosinophils, pre-B-cell precursors, proliferation of Natural Cytotoxic (NC) cells but not Natural Killer (NK) cells and to promote the formation of osteoclasts.

Il-3 also stimulates the effector functions of monocytes, eosinophils and basophils, thereby having the potential to regulate inflammation and allergy (Elliott *et al*., 1990; Haak-Frendesco *et al*., 1988; Lopez *et al.*, 1990). Human endothelial cells express the IL-3 receptor which expression is enhanced by tumor necrosis alpha (TNFa). Il-3 stimulation of TNFa-activated endothelial cells enhanced IL-8 production, E-selectin expression and neutrophils transmigration (Korpelainen *et al*., 1993). This suggests that IL-3 can play a role in inflammation not only by stimulating effector functions of mature leukocytes but also regulating their localization to sites of inflammation through its action on endothelium.

Pulaski (Pulaski *et al.*, 1993) transfected an alveolar lung cell cacinoma of the mouse (tumor line 1) with IL-2 or IL-3 genes. Both transfectants were less tumorgenic than the parent line 1 as manifested by slower growth, no growth or initial growth and rejection. The TIL isolated from the transfectants showed a strongly increased cytotoxic response to line 1 tumor cells. This was CD8 dependent in the case of the IL-2 transfectant, but CD8 and CD4 dependent in the case of the IL-3 transfectant. The same dependency was demonstrated for *in vivo* decreased tumorgenicity. The MHC class I expression was increased in the IL-3 transfected line 1 tumors.

McBride et al (McBride *et al*., 1993) studied the effect of transfection with IL-3 gene of a moderately and a non-immunogenic mouse fibrosarcoma on the immunogenicity of irradiated tumor cell vaccins as measured by the development of protection to parental tumor growth. In comparison to other cytokines, IL-3 was surprisingly effective in particular with the non immunogenic tumor. Protection was tumor specific and could be transferred with spleen cells from immunized mice. Both transfectants showed increased MHC I expression. Again these studies do not disclose the importance and the significant effect o0f the high initial dose followed by the sustained release as the present invention shows.

The invention further provides a composition as disclosed above further comprising an adjuvans. The adjuvans of course further enhances the immunogenicity of the compositions in general and specifically when the adjuvans comprises inactivated cells, for instance cracked cells. A very suitable way to arrive at a composition comprising an initial burst dose of a cytokine, adenoviruses providing a source for a sustained production of the cytokine and the cellular adjuvans is culturing adenovirusproducer cells which express the cytokine, letting these cells express a cytokine and thus produce a lot of recombinant adenovirus and inactivating said cells so that virus and cytokine can be released. The injection of the composition such as adenoproducer cells is a new delivery system to obtain high levels of inflammatory cytokines locally at the tumor site and continuous production of non-toxic levels of the cytokines by the tumor cells.

The invented system provides 1) a relatively high inital dose of the cytokine protein, 2) recombinant adenoviruses that transduce tumor cells and are thus responsible for continuous production of cytokines by the tumor cells and 3) foreign cellular proteins that act as an adjuvant in the induction of the inflammation and immune response against the tumor cells. Alternatively, the adjuvant may be replaced by compositions that act as adjuvants, such as Freund's adjuvant, BCG, etc.
As an preferred example of the compositions according to the invention, cracked cells are presented. This includes the delivery of adenovirus infected cells that are frozen after virus infection.

Briefly, recombinant adenovirus replicates in adenovirus packaging cells. In our examples we use E1 deleted adenovirus vectors. Recombinant adenovirus vectors harbouring the rat or human IL-3 or the human IL-1a cDNA are deleted for the E1 region, as indicated in Table I. In these vectors, the cytokine gene is either driven by the adenovirus-2 derived Major Late Promoter (MLP) or by the Cytomegalovirus Immediate Early promoter (CMV). As a control, an adenovirus vector harboring Herpes Simplex Virus thymidine kinase gene (a non-cytokine gene), was used. The deletion of the E1 region makes these resulting adenovirus vectors replication defective. In order to be able to produce these vectors, they are propagated on cell lines that harbor the E1 region of adenovirus, such as 293 (Graham *et al*., 1977), 911 (Fallaux *et al.*, 1996) or PER.C6 cells (Fallaux, 1996). In our examples, cracked cells mean 293 cells that were infected with recombinant adneovirus and that were frozen two days after infection with adenovirus.

Upon thawing, the cells are lysed and therefore the cytokines present intra-cellularly are released. In a productive adenovirus infection, the levels of the cytokines produced are high (see experimental) if the cytokine gene is driven by a promoter that is active in the adenovirus producer cells. In addition to release of the cytokine, adenovirus vectors are released. If the adeno-producer cells are lysed just before the onset of full CPE or at full CPE, high titers of the adenovirus vector are obtained (see example 1). The cracked cells are injected, e.g. intratumorally. The use of cracked cells overcomes a number of the limitations of using living cells. Living cells are also adenovirus producer cells, but in this procedure the cells are harvested e.g. 48 hours after infection with adenovirus and injected without cracking or other inactivation of the cells.

First, application of living cells is logistically difficult (note that living cells are required 'Just In Time'). Second, it is not possible to test the infected living cells for safety parameters, as the living cells have to be applied immediately. Third, the living adenoproducer cells can not be characterized (e.g. for titer, protein, cytokine levels) at the moment of injection, but only retrospectively. Fourth, availability of living adenoproducer cells is poor.

On the other hand, large batches of cracked cells are produced in advance and stored frozen, allowing the treatment of many subjects at the same time with identical material, thus excluding influence of variations in virus titers or cytokine concentrations.

Similarly large batches of the compositions according to the invention can be prepared by mixing the individual components.

The invention further provides a method for the treatment of malignancies comprising contacting said malignancies with a composition according to the invention, in particular for the treatment of solid tumors.

Compositions of cytokine proteins, recombinant adenovirus vectors and adjuvant, such as cracked cells will be used in the clinic for the treatment of patients with cancer, including but not limited to non-small cell lung cancer, small cell lung cancer, melanoma, breast cancer.

The composition will be injected directly into the tumor, either in the primary tumor or in metastases. Most likely, a number of injections are needed for optimal therapeutic results.

In addition, this treatment may be extended as follows. Tumors (or samples of it) treated with the composition will be removed surgically from the patient (for a representative animal experiment, see example 4). The tumor cells in the samples will then be killed, e.g. by freezing of the tumor mass or by irradiation. Then, mixtures of 'mashed tumor cells' will be used as a 'vaccine'. They will be applied to the patient, either intra-tumorally or systemically (intra-venous, subcutaneous, intra-peritoneal, intra-muscular).

Alternatively, mashed tumor cells may be added to other patients bearing similar tumors as well.

Furthermore, tumor cell lines, either being cultured from tumor samples obtained after surgery or being available as established cell lines, will be irradiated and co-injected with the composition. The adenovirus vectors present in the composition will infect the irradiated tumor cells, that will in turn produce cytokine until they die because of the irradiation. The high levels of cytokine present in the composition, together with the adjuvant and the cytokine producing tumor cells will function as a cancer vaccine, either prophylactic of for the treatment of diagnosed cancer.

In addition, effector cells infiltrating the tumormass after delivery of the composition, such as e.g. Tumor Infiltrating Lymphocytes, will be isolated from the tumors, expanded *ex vivo* and re-injected into the patient. E.g. Tumor Infiltrating Lymphocytes will then home to tumor cells and subsequently lyse tumor cells.
Thus the invention also provides a method further comprising inactivating treated cells from the solid tumor, mixing them with a suitable adjuvans and administrating said mixture to the mammal from which the tumor cells originate, as well as a vaccine for the treatment of solid tumors comprising inactivated autologous tumor cells, recombinant adenovirus, a cytokine or a functional equivalent or fragment thereof and an adjuvans.

### Experimental.

### Example 1.

### Cloning of human IL-1α and rat interleukin-3 in adenovirus vectors

The cloning, sequence analysis and generation of adenovirus vectors has been described in detail in patent application EP 95 20 2213 incorporated herein by reference.
The respective adenovirus vectors are deleted for the E1, but the E3 region was retained in these vectors. The cytokine genes are driven by either the adenovirus-2 derived Major late promoter (MLP) or the Cytomegalovirus promoter (CMV). The names of the viruses are IG.Ad.MLP.hIL-1α, IG.Ad.MLP.rIL-3 and IG.Ad.CMV.rIL-3 (see Table I).
Recombinant adenovirus IG.Ad.CMV.rIL-3 has been deposited at the ECACC under accession number V96071634.
The generation and propagation of these vectors on E1 complementing cell lines has been described in patent application EP 95 20 2213. Propagation on 911 cells or PER.C6 is described by (Fallaux et al., 1996).

### Preparation of adenoproducer cells.

The 293 cell line is a human embryo kidney cell line expressing adenovirus type 5 (Ad5) E1A and E1B functions (Graham et al., 1977). They were cultured in Dulbecco's modified Eagle's medium supplemented with peniciline (100 IU/ml; Gibco), streptomycine (50 mg/ml; Gibco), 10% fetal calf serum and non-essential amino acids, at 37^{°}C and 5% CO2.
To prepare adenoproducer cells, 293 cells, at 70-80% confluency, are infected with recombinant adenovirus at an m.o.i. of 10. After 48 hours, the infected cells are trypsinized and suspended in PBS. The cells are stored at -20°C for future injections ("cracked" cells).
The cracked cells are titered to determine the amount of virus particles and the levels of the cytokine. Adenovirus titration was performed by End Point CPE assay (Precious and Russell, 1985) on 911 cells (Fallaux et al., 1996).
The amount of rat IL-3 present in the adenoproducer cells at the moment of injection was quantitated in a FDC-P1 proliferative assay (Coligan et al., 1991). Supernatant of A549 cells (human non-small cell lung carcinoma) infected with IG.Ad.CMV.rIL-3 are used as rat IL-3 standard. The units of rat IL-3 of the standard solution were previously determined in a colony forming assay on rat bone marrow cells (Merchav and Wagemaker, 1984). 1 unit of rat IL-3 was defined by the concentration that induced 50% maximum number of colonies.
The human IL-1α protein present in the adenoproducer cells at the moment of injection is quantitated by an ELISA (R&D systems, Netherlands) after sonication of 293 cells.

### Levels of cytokines produced in adenovirus producer cells compared to non-producer cells

Samples of IG.Ad.MLP.hIL-1α -293 cells prepared as described above were used to determine the amount of cytokines present in the producer cells at the moment of injection.
Lysates of the adenoproducer cells were prepared by sonication for 45 seconds and centrifugation at 14000 rpm for 5 minutes. The supernatants were assayed as follows: for human IL-1α, using an ELISA (R&D systems, Netherlands).
The quantification of human IL-1α in 5 samples revealed that the concentration of protein is about 77 ng ± 28.2 ng per 10⁷ cells.
Table I compared the production of IL-1α protein in IG.Ad.MLP.hIL-1α -293 cells with L42 tumor cells infected with IG.Ad.MLP.hIL-1α.

IG.Ad.MLP.hIL-1α-293 cells, 48 hrs after infection (moi 10)
L42 cells infected with IG.Ad.MLP.hIL-1α, 72 hrs after infection (moi 10) 77 ng/10⁷ cells
0.7 ng/ 10⁷ cells

### Determination of virus titers

Samples of 293 cells producing either IG.Ad.MLP.hIL-1a or IG.Ad.CMV.rIL-3 were stored at -20 ^{°}C to later determine the virus concentration present in the producer cells at the moment of injection. The samples underwent three freeze/thaw cycles, were centrifuged at 6000rpm during 5 minutes and the supernatant containing the virus particles was titrated in an End Point CPE assay on 911 cells.
Seven different samples of IG.Ad.MLP.hIL-1α-293 producers were used to determine the number of infectious particles contained in 10⁷ cells. The average was 1x10¹⁰ infectious particles per 10⁷ cells.
In the case of IG.Ad.CMV.rIL-3-293 producers 14 samples were examined. The average number of infectious particles present in 10⁷ cells was 3x10¹⁰.

### Example 2.

### Differential protein release kinetics after intratumoral injection of living or cracked adenoproducer cells.

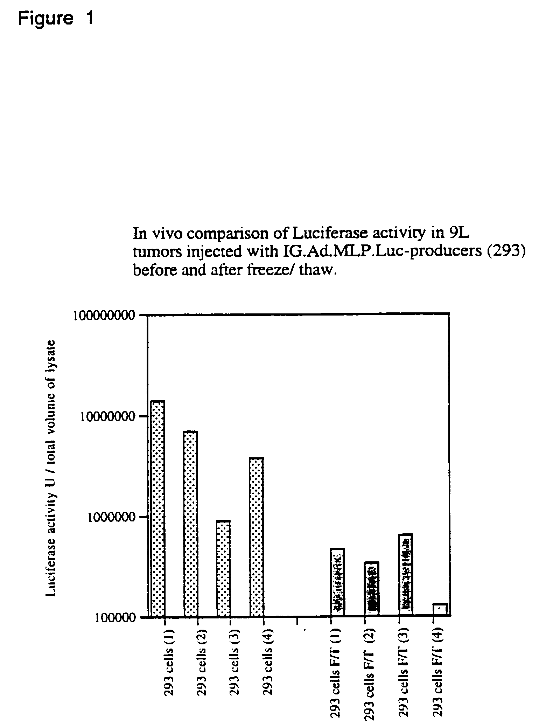

The *in vivo* gene expression after intratumorally injection of living or cracked adenoproducer was compared using luciferase as marker gene. For this purpose, 9L tumor (Weizsaecker et al., 1981) pieces were implanted sc. in the left flank of F344 rats (n=8). IG.Ad.MLP.Luc-293 producers were prepared as it was described previously. 48 hours after virus infection, the producers were harvested and divided in 8 independent samples of 10⁷ cells/300µl of PBS. Four samples were directly injected in sc 9L tumors 11 days after tumor implantation. The rest of the samples were injected into 9L tumors after being frozen in solid CO² and thawed at room temperature. Three days later the rats were sacrified, the tumors were removed and luciferase activity was determinated as described by (Fortunati et al., 1996). The luciferase activity was expressed in amount of luciferase units per total volume of tumor lysate (Figure 1). Three days after injection, the luciferase activity detected in tumor lysates was higher in the tumors injected with living cells producing IG.Ad.MLP.luc as compared to the injected with cracked cells producing the same recombinant vector. The activity observed in the tumors injected with cracked cells corresponds to expression of the luciferase gene after adenovirus infection of tumor cells. The difference observed in the living cells injected tumors is mainly caused by the 293 cells that still remain in the tumor tissue and continue producing recombinant adenovirus that can infect tumor cells and increase the luciferase activity. From these results we conclude that using living cells, expression over a longer period of time is achieved, whereas in the case of cracked cells, after the freeze/thaw cycle the cells lyse and release immediately free cytokine and recombinant adenovirus. This burst of cytokines can be an important characteristic of the "cracked" system considering the hypothesis that high levels of IL-1 or IL-3 are necessary. However, as these molecules have a short half life (e.g. IL-3 (40 min.) (Crepper et al., 1984), continuous production may be a pre-requisite to maintain certain local levels of the cytokine. This can be achieved by the transduction of tumor cells by the adenovirus vectors provided by the cracked cells.

### Example 3

### Tumor regression after administration of cracked adenoproducers to rat tumors.

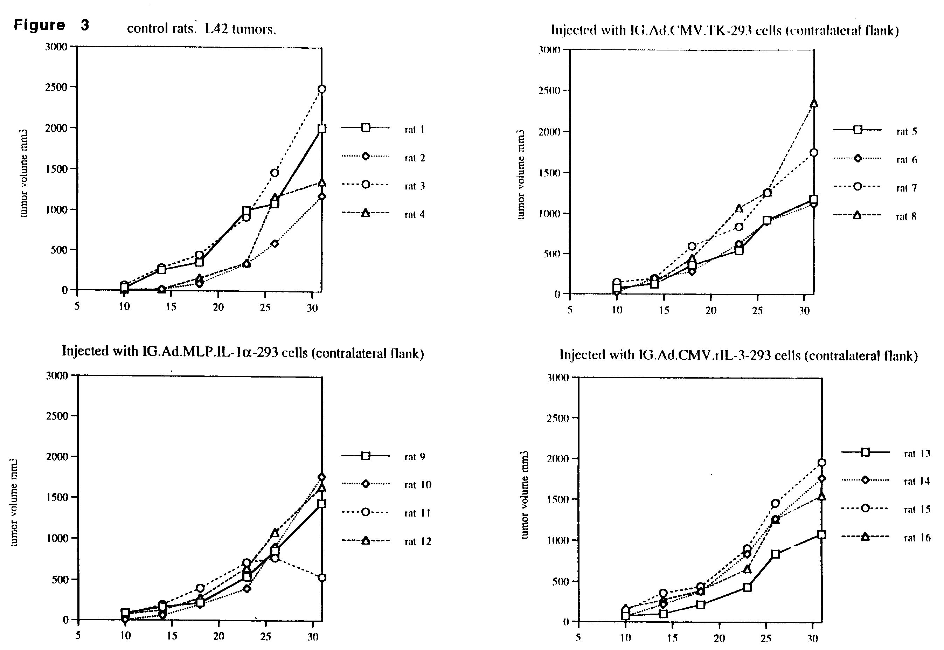

We were interested in studying the possibility to elicite an immune reaction against established tumors after delivery of high concentration of inflammatory cytokines like IL-1 and IL-3. The tumor model used was the L42 rat NSCLC (non small cell lung cancer) in the WAG/Rij rat strain (Kal et al., 1986; Kal et al., 1991). This is a non-immunogenic animal model, as vaccination of rats with irradiated tumor cells did not protect against a challenge with parental tumor cells.
The response was monitored following the growth rate of injected tumors and also distant non-treated tumors that imitated metastases. The protocol was as follow:
on day 1: subcutaneous implantation of L42 tumor pieces bilateral.
on day 10: "cracked" adenoproducer cells were injected into the left tumor, daily during 10 days starting on day 10. The rats were divided into four groups, each group containing 4 animals:
   1- non treated animals bearing L42 tumors.
   2- animals injected daily with 10⁷ IG.Ad.MLP.hIL-1α-293 producer cells.
   3- animals injected daily with 2.5x10⁵ IG.Ad.CMV.rIL-3-293 producer cells.
   4- animals injected daily with 10⁷ IG.Ad.CMV.TK-293 producer cells.
Initial tumor volume: 150-200 mm³ approx. The sizes of the tumors in both flanks were measured twice a week.

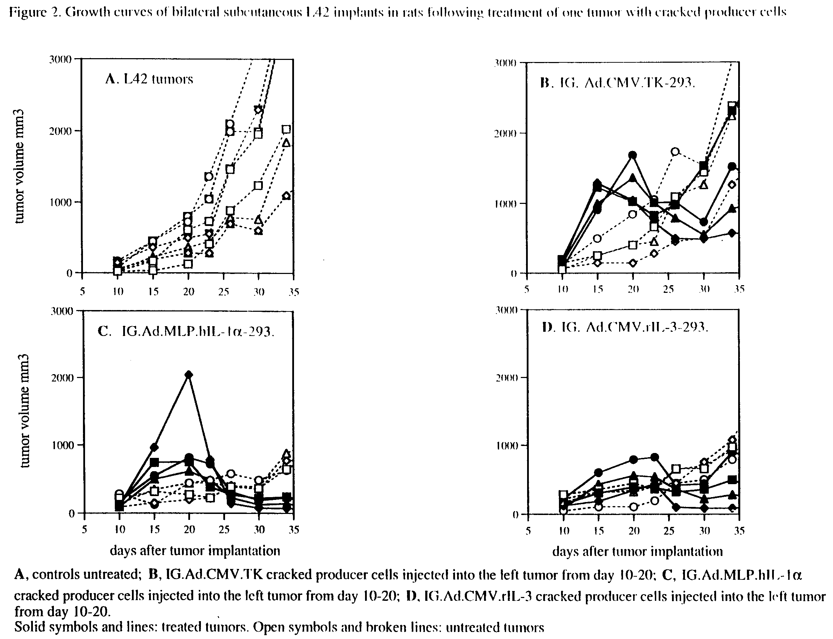

Figure 2 shows the growth curves of the subcutaneous tumors in the different groups. The results demonstrate that repeated intratumoral injections of cracked adeno-cytokine producer cells induce partial responses in the treated as well as in the untreated contralateral tumor, reflected as a growth delay of the tumors in the cytokine groups comparing to the control groups (non treated or injected with IG.Ad.CMV.TK-293 cells).
To study which cells invade the tumor and induce immunogenicity, an experiment was performed following the same protocol described above. Rats from different groups were sacrified at day 16 (n=2) and day 24 (n=2). The histological examination of the ipsilateral and contralateral tumors revealed differential features in the cytokine groups comparing to the control groups. At day 16, an increase infiltration of stroma with mononuclear, macrophages and granulocytes with focus of necrosis were observed in the treated tumors of rats injected with the "cracked"-cytokines cells. In these rats, at day 24, most of the ipsilateral tumor was necrotic with scar tissue and small areas of living tumor cells while the contralateral tumor presented the identical picture described for the injected tumor on day 16.
To investigate if the antitumor effect observed was due to high cytokines levels in the circulation, "cracked" cells were injected subcutaneously and the growth of an unilateral contralateral tumor was monitored. No difference in tumor growth was observed in the cytokine groups comparing to the control groups (Figure 3).
Therefore, we have observed that IL-1α and IL-3 provided in high local concentrations by intratumoral injection of "cracked" cells are similarly active in inducing regression of treated and untreated distant tumors. This effect is therefore immune mediated and probably not due to a systemic effect of the interleukin leaking out of the tumor.

### Example 4

### Application of the "cracked" adenoproducer cells: Therapeutic vaccination using tumor cell lysates previously treated with "cracked" adenoproducer cells.

The use of cracked adenoproducer cells allows us to reach the required high level of interleukins inside the tumor to obtain therapeutic responses as described in the previous experiment. We wanted to investigate the mechanism of action of the mentioned inflammatory cytokines. Our working hypothesis is that the inflammatory reaction induced by IL-1α or IL-3 in the treated tumor liberates tumor antigens in a form that can be presented to elicit tumor specific CTL. These cells will then via the circulation attack the contralateral non treated tumor cells and cause regression at a distance.
To explore this theory, animals bearing unilateral sc L42 tumors, were treated with cracked cells intratumorally daily, starting 15 days after tumor implantation. The rats were divided in four groups:
1- animals with non treated L42 tumors.
2- animals injected with 293 cells infected with IG.Ad.CMV.TK (10⁷ cells/injection)
3- animals injected with 293 cells infected with IG.Ad.MLP.hIL-1a (10⁷ cells/injection).
4- animals injected with 293 cells infected with IG.Ad.CMV.rIL-3 (2.5x10⁵ cells/injection).
At two stages during the cracked treatment of the tumor (after the 4th and 8th injection), the rats were sacrified and standard pieces of tumor tissue were taken to prepare a tumor cell lysate.
Equal tumor tissue from the two different time points were used together to vaccinate rats bearing unilateral sc L42 tumors (10 days after tumor implantation). These rats were divided again in the same groups described above and received the vaccination either subcutaneous (n=4) or intraperitoneal (n=3). The vaccination was performed two times with an interval of 7 days between injections.
The size of the subcutaneous tumor was measured twice a week.

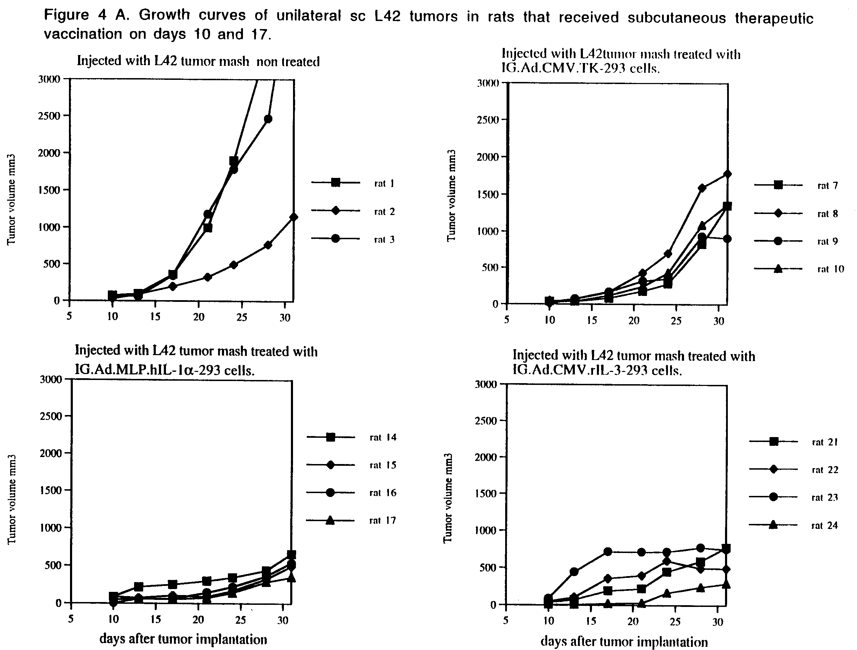

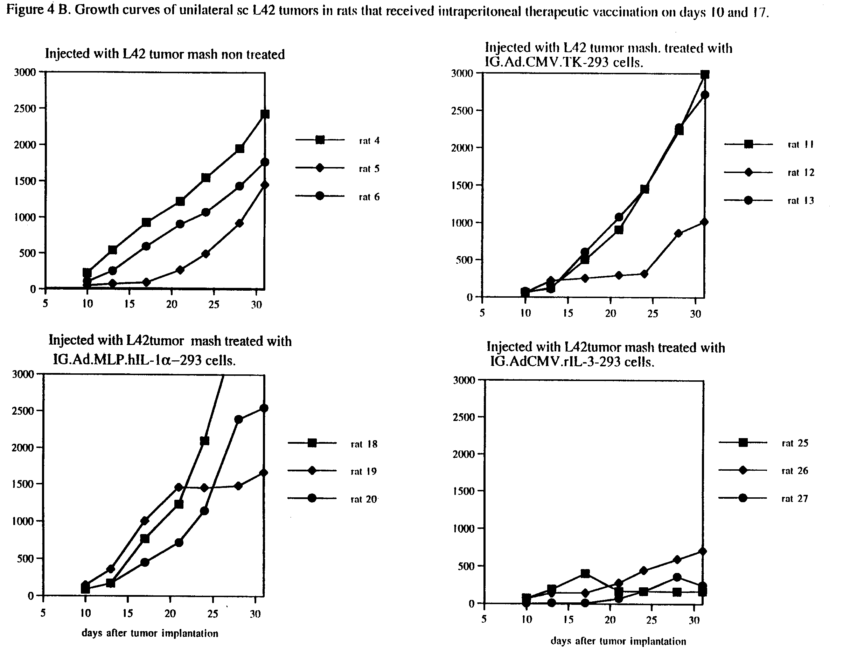

Figures 4A and 4B show the growth curves of the tumors after the therapeutic vaccination. Tumor regression was observed after subcutaneous vaccination with tumor mashes treated either with IG.Ad.MLP.hIL-1α-293 cells or with IC.Ad.CMV.rIL-3-293 cells. The intraperitoneal vaccination was effective only in the case of IG.Ad.CMV.rIL-3-293 cells.

### Experiments to determine which of the components is responsible for the observed results.

In order to determine whether either one of the components present in the cracked cells is responsible for the observed therapeutic effects, or that all components of the compositions according to the invention are required for full effectivity, the following experiments are performed.
The first set of experiments aims at assessing which of the components is active in the cracked cell lysate include direct injection of adenovirus vector preparation.
We have demonstrated that direct single injection of recombinant adenovirus harboring either hIL-1α or rIL-3 was not effective for treatment of L42 tumors (see patent application EP 95 20 2213). Experiments will be performed, where multiple doses of the adenovirus will be injected, exactly according to the scheme that was followed for the injection of cracked cells This means, that doses of adenovirus equal to the doses of adenovirus present in the cracked cells, will be injected for 10 consecutive days, as was performed for cracked cells.
The second series of experiments make use of differential inactivation of adenovirus and rIL-3. Mouse IL-3 is known to be resistant to low pH and temperature (Ihle, 1989), whereas adenovirus particles are sensitive to low pH and heat inactivation. In addition, adenovirus particles can be inactivated completely by UV irradiation (Cotten et al., 1992). The amino-acid sequence homology between rat and mouse IL-3 is known to be only 54% (Cohen et al., 1986). Therefore, we first determine the temperature sensitivity of rat-IL-3 in cracked cell preparations, by incubating it at increasing temperatures and to determine the bioactivity of IL-3 as a function of the temperature. In parallel, we determine the virus titer as a function of the temperature in the same preparations. The temperature where adenovirustiters have dropped at least 3 logs but where the IL-3 bioactivity is fully retained is used to inactivate other batches of cracked cells, that are injected into subcutaneous L42 rat tumors *in vivo* cells by giving 10 daily injections, as described before. Anti-tumor activity is monitored by assessing the tumor volumes. If antitumor activity is measured, than intact IG.Ad.CMV.rIL-3 recombinant adenovirus particles are not the sole active components in the cracked cells.
A similar experiment is performed by changing the pH of cracked cells solution.
rIL-3 activity and adenovirus titer in the preps are determined as a function of pH.
Batches with at least a 3 log decreases adenovirus titer but fully biologically active rIL-3 are tested for anti-tumor acitivity as described above. A third way of selective inactivation of adenovirus is by UV inactivation. Adenovirus particles can be inactivated completely by UV irradiation. This method is applied to inactivate adenovirus particles in cracked cell preparations. Such inactivated batches are tested for rIL-3 biologically active rIL-3 and batches where the activity was fully retained are tested for in vivo anti-tumor activity as described above.
The methods described all aim at inactivation of the adenovirus particles. They will provide us with data whether the virus, the high initial cytokine dosis or the combination of these two components is mainly responsible for the observed anti-tumor effects

### References.

Apte, R. N., Douvdevani, A., Zoller, M., and White, R. M. (1994a): Immune recognition and rejection of IL-1 alpha gene transduced tumor cells., pp. 97-112. In G. Forni, R. Foa, A. Santoni, and L. Frati (Eds): Cytokine-induced tumor immunogenicity, Acad. Press, London.
Apte, R. N., Douvdevani, A., Zoller, M., White, R. M., Dvorkin, T., Shimoni, N., Huleihel, M., Fima, E., Hacham, M., and Voronov, E. (1994b): Involvements of immune responses in the eradication of IL-1 alpha gene-transduced tumor cells: mechanisms of tumor rejection and immunotherapeutical implications. Folia Biol. Praha 40, 1-18.
Berk, A. J. (1986): Adenovirus promoters and E1A transactivation. Ann. Rev. Genet. 20, 45-79.
Blaese, M., Blankenstein, T., Brenner, M., Cohen-Haguenauer, O., Gansbacher, B., Russell, S., Sorrentino, B., and Velu, T. (1995): Vectors in cancer therapy: how will they deliver? Cancer Gene Ther. 2, 291-297.
Blankenstein, T., Qin, Z., Uberla, K., Muller, W., Rosen, H., Volk, H. D., and Diamantstein, T. (1991): Tumor supression after tumor cell-targeted tumor necrosis alpha gene transfer. J. Exp. Med. 173, 1047-1052.
Blazar, B. R., Widmer, M. B., Taylor, P. A., and Vallera, D. A. (1992): Promotion of a murine marrow alloengraftment and hematopoietic recovery across the major histocompatibility barrier by administration of recombinant human interleukin-1 alpha. Blood 80, 1614-1622.
Bout, A., Imler, J. L., Schultz, H., Perricaudet, M., Zurcher, C., Herbrink, P., Valerio, D., and Pavirani, A. (1994a): In vivo adenovirus-mediated transfer of human CFTR cDNA to rhesus monkey airway epithelium: efficacy, toxicity and safety. Gene Ther., in press.
Bout, A., Perricaudet, M., Baskin, G., Imler, J. L., Scholte, B. J., Pavirani, A., and Valerio, D. (1994b): Lung gene therapy: in vivo adenovirus-mediated gene transfer to rhesus monkey airway epithelium. Hum. Gene Ther. 5, 3-10.
Brody, S. L., and Crystal, R. G. (1994): Adenovirus-mediated in vivo gene transfer. Ann N Y Acad Sci 716, 90-101.
Cohen, D. R., Hapel, A. J., and Young, I. G. (1986): Cloning and expression of the rat interleukin-3 gene. Nucl. Acids Res. 14, 3641-3658.
Coligan, J. E., Kruisbeek, A. M., Margulies, D. H., Shevach, E. M., and Strober, W. (1991): Current protocols in immunology. Greene and Wiley-Interscience. New York.
Colombo, M., Ferrari, G., Stoppacciaro, A., Parenza, M., Rodolfo, M., Mavilio, F., and Parmiani, G. (1991): Granulocyte colony-stimulating factor gene transfer supresses tumorigenicity of a murine adenocarcinoma in vivo. J. Exp. Med. 173, 889-897.
Cotten, M., Wagner, E., Zatloukal, K., Phillips, S., Curiel, D. T., and Birnstiel, M. L. (1992): High efficiency receptor-mediated delivery of small and large (48 kb) gene constructs using the endosome disruption activity of defective of chemically inactivated adenovirus particles. Proc. Natl. Acad. Sci. USA 89, 6094-6098.
Crepper, R. M., Clark-Lewis, I., and Schrader, J. W. (1984): Immunology 53, 33-42.
Douvdevani, A., Huleihel, M., Zöller, M., Segal, S., and Apte, R. N. (1992): Reduced tumorigenicity of fibrosarcomas which constitutively generate Il-1a either spontaneously or following Il-1α gene transfer. Int. J. Cancer 51, 822-830.
Dranoff, G., Jaffee, E., Lazenby, A., Golumbek, P., Levitsky, H., Brose, K., Jackson, V., Hamada, H., Pardoll, D., and Mulligan, R. C. (1993): Vaccination with irradiated tumor cells engineered to secrete murine granulocyte-macrophage colony-stimulating factor stimulates potent, specific and long-lasting anti-tumor immunity. Proc. Natl. Acad. Sci. 90, 3539-3543.
Elliott, J. I., Vadas, M. A., Cleland, L. G., Gamble, J. R., and Lopez, A. F. (1990): IL-3 and granulocyte-macrophage colony-stimulating factor simulate distinct phases of adhesion in human monocytes. J. Immunnol. 145, 167.
Fallaux, F. J. (1996): Gene Therapy for hemophila A: Towards the use of adenovirus vectors? University of Leiden. Leiden.
Fallaux, F. J., Kranenburg, O., Cramer, S. J., Houweling, A., Ormondt, H. v., Hoeben, R. C., and Eb, A. J. v. d. (1996): Characterization of 911: a new helper cell line for the titration and propagation of early-region-1-deleted adenoviral vectors. Hum. Gene Ther. 7, 215-222.
Fearon, E. R., Pardoll, D. M., Itaya, T., Golumbek, P., Levitsky, H. I., Simons, J. W., Karasuyama, H., Vogestein, B., and Frost, P. (1990): Interleukin-2 production by tumor cell bypasses T helper function in the generation of an antitumor response. Cell 60, 397-401.
Ferrantini, M., Proietti, E., Santodonato, L., Gabriele, L., Peretti, M., Plavec, I., Meyer, F., Kaido, T., Gresser, I., and Belardelli, F. (1993): a1-Interferon gene transfer into metastatic friend leukemia cells abrogate tumorigenicity in immunocompetent mice: Antitumor therapy by means of interferon-producing cells. Cancer Res. 53, 1107-1112.
Fortunati, E., Bout, A., Zanta, M. A., Valerio, D., and Scarpa, M. (1996): in vitro and in vivo gene transfer to pulmonary cells mediated by cationic liposomes. Biochim. Biophys. Acta in press.
Fung, M. C., Hapel, A. J., Ymer, S., Cohen, D. R., Johnson, R. N., Campbell, H. D., and Young, I. G. (1984): Molecular cloning of cDNA for mouse interleukin-3. Nature 307, 233.
Gansbacher, B., Bannerji, R., Daniels, B., Zier, K., Cronin, K., and Gilboa, E. (1990a): Retroviral vector mediated gamma-interferon gene transfer into tumor cells generates potent and long lasting antitumor immunity. Cancer Res. 50, 7820-7824.
Gansbacher, B., Zier, K., Daniels, B., Cronin, K., Bannerji, R., and Gilboa, E. (1990b): Interleukin-2 gene transfer into tumor cells abrogates tumorigenicity and induces protective immunity. J. Exp. Med. 172, 1217.
Golumbek, P., Lazenby, A., Levitsky, H. I., Jaffee, L. M., Karasuyama, H., Baker, M., and Pardoll, D. M. (1991): Treatment of establish renal cancer by tumor cells engineered to secrete interleukin-4. Science 254, 713-716.
Graham, F. L., Smiley, J., Russell, W. C., and Nairn, R. (1977): Characteristics of a human cell line transformed by DNA from adenovirus type 5. J. Gen. Virol. 36, 59-72.
Haak-Frendesco, M., Arai, N., Arai, K. I., Baeza, M. L., Finn, A., and Kaplan, A. P. (1988): Human recombinant granulocyte-macrophage colony stimulating factor and interleukin-3 cause basophil histamine release. J. Clin. Invest. 82, 17.
Heufler, C., Koch, F., and Schuler, G. (1988): Granulocyte-macrophage colony-stimulating factor and interleukin-1 mediate the maturation of murine epidermal langerghans cells into potent immunostimulatory dendritic cells. J. Exp. Med. 167, 700-705.
Hock, H., Dorsch, M., Diamantstein, T., and Blankenstein, T. (1991): Interleukin-7 induces CD4+ T cell dependent tumor rejection. J. Exp. Med. 174, 1291-1298.
Ihle, J. N. (1989): The molecular biology of interleukin-3, pp. 59-102. In J. M. Cruse, and R. E. Lewis (Eds): Immunoregulatory cytokines and cell growth, Karger, Basel.
Ihle, N. I., Keller, J., Oroszlan, S., Henderson, L. E., Copeland, T. D., Fitch, F., Prystowsky, M. B., Goldwasser, E., Schrader, J. W., Palaszynski, E., Dy, M., and Lebel, B. (1983): Biologic properties of homogeneous interleukin-3: Demonstration of WEHI-3 growth factor activity, mast cell growth factor activity, P cell stimulating factor activity, colony-stimulating factor activity, and histamine producing cell-stimulating factor activity. J. Immunol. 131, 282.
Kal, H. B., Berkel, A. H. v., Zurcher, C., Smink, T., and Bekkum, D. W. v. (1986): A rat lung cancer modes based on intra-pulmonary implantation of tumour material. Radiother. and Oncol. 6, 231-238.
Kal, H. B., Meijnders, P. J. N., Berkel, A. H. v., and Bekkum, D. W. v. (1991): Response to chemotherapy of non-small cell bronchial rat tumours growing subcutaneously or in the lung. In vivo 5, 301-306.
Kindler, V., Thorens, B., Kossodo, S. d., Allet, B., Eliason, J. F., Thatcher, D., Farber, N., and Vassali, P. (1986): Stimulation of hematopolesis in vivo by recombinant bacterial murine interleukin-3. Proc. Natl. Acad. Sci. USA 83, 1001.
Kita, H., Ohnishi, T., Okubo, Y., Weiler, D., Abrms, J. S., and Gleich, G. J. (1991): Granulocyte/macrophage colony-stimulating factor and interleukin-3 release from human peripheral blood eosinophils and neutrophils. J. Exp. Med. 174, 754.
Konishi, T., Hiraishi, M., Mafune, K., Miyama, T., Hirata, T., Mori, K., Nishina, H., and Idezuki, Y. (1994): Therapeutic efficacy and toxicity of sequential methotrexate and 5-fluorouracil in gastric cancer. Anticancer-Res 14, 1277-9 issn: 0250-7005.
Korpelainen, E. I., Gamble, J. R., Smith, W. B., Goodall, G. J., Qiju, S., Woodcock, J. M., Dottore, M., Vadas, M., and Lopez, A. F. (1993): The receptor for interleukin 3 is selectively induced in human endothelial cells by tumor necrosis factor a and potentiates interleukin 8 secretion and neutrophil transmigration. Proc. Natl. Acad. Sci USA 90, 11137.
Lopez, A. F., Eglinton, J. M., Lyons, A. B., Tapley, P. M., To, L. B., Park, L. S., Clark, S. C., and Vadas, M. A. (1990): Human interleukin-3 inhibits the binding of granulocyte- macrophage colony stimulating factor and interleukin-5 to basophils and strongly enhances their functional activation. J. Cell Physiol. 145, 69.
McBride, W. H., Dougherty, G. D., Wallis, A. E., Economou, J. S., and Chiang, C. S. (1993): Interleukin-3 in gene therapy of cancer. Folia Biologica (Praha) 40, 62-73.
McBride, W. H., Thacker, J. D., Comora, S., Economu, J. S., Kelley, D., Hogge, D., Dubinnet, S. M., and Dougherty, G. J. (1992): Genetic modification of a murine fibrosarcoma to produce interleukin-7 stimulates host cell infiltration and tumor immunity. Cancer Res. 52, 3931-3937.
Merchav, S., and Wagemaker, G. (1984): Int. J. Cell Cloning 2, 356.
Nakata, K., Kashimoto, S., Yoshida, H., Oku, T., and Nakamura, S. (1988): Augmented antitumor effect of recombinnat human interleukin-1 alpha by indomethacin. Cancer Res. 48, 584-588.
Niemeyer, C. M., Sieff, C. A., mathey-Prevot, B., Wimperis, J. Z., Bierer, B. E., Clark, S. C., and Nathan, D. G. (1989): Expresison of human interleukin-3 (multi-CSF) is restricted to human lymphocytes and T cell tumor cell lines. Blood 73, 945-951.
Platzer, C., Richter, G., Uberla, K., Hock, H., and Blankenstein, T. (1992): Interleukin-4-mediated tumor supression in nude mice involves interferon-gamma. Eur. J. Immunol. 22, 1729-1733.
Precious, B., and Russell, W. C. (1985): Growth, purification and titration of adenoviruses, pp. 157-167. In B. Mahy (Ed.): Virology: a practical approach, Raven Press, Ltd, New York.
Pulaski, B. A., Mc Adam, A. J., Hutter, E. K., Biggar, S., Lord, E. M., and Frelinger, J. G. (1993): Interleukin-3 enhances development of tumor-reactive cytotoxic cells by a CD4-dependent mechanism. Cancer Res. 53, 2112-2117.
Schrader, J. W., Clark-Lewis, I., R.B.Ccrapper, Leslie, K. B., Schrader, S., Vargios, G., and Ziltener, H. J. (1988): Interleukin-3: The panspecific Hemopoietin. In J. W.
Schrader (Ed.): Lymphokines, Academic Press, San Diego.
Schwartz, G. N., Neta, R., Vinculle, R. M., Patchen, M. L., and Mac Vittie, T. J. (1988): Recovery of hematopoietic colony-forming cells in irradiated mice pretreated with interleukin-1 (IL-1). Exp.Hematol. 16, 752-757.
Smith, A. E. (1995): Viral vectors in gene therapy. Ann. Rev. Microbiol. 49, 807-838.
Stratford-Perricaudet, L., and Perricaudet, M. (1991): Gene Transfer into Animals: the promise of adenovirus., pp. 51-61. In O. Cohen-Adenauer, and M. Boiron (Eds): Human gene transfer, INSERM.
Tahara, H., Zeh III, H. J., Storkus, W. J., Pappo, I., Watkins, S. C., Gubler, U., Wolf, S. F., Robbins, P. D., and Lotze, M. T. (1994): Fibroblasts genetically engineered to secrete interleukin-12 can suppress tumor growth and induce antitumor immunity to a murine melanoma in vivo. Cancer Res. 54, 182-189.
Tooze, J. (1981): DNA Tumor Viruses (revised). Cold Spring Harbor Laboratory. Cold Spring Harbor, New York.
Vincent, A. J. P. E., Esandi, M. d. C., Someren, G. D. v., Noteboom, J. L., C.J.J, A., Vecht, C., Smitt, P. A. E. S., Bekkum, D. W. v., Valerio, D., Hoogerbrugge, P. M., and Bout, A. (1996a): Treatment of Lepto-meningeal metastasis in a rat model using a recombinant adenovirus containing the HSV-tk gene. J. Neurosurg. in press.
Vincent, A. J. P. E., Vogels, R., Someren, G. v., Esandi, M. d. C., Noteboom, J. L., Avezaat, C. J. J., Vecht, C., Bekkum, D. W. v., Valerio, D., Bout, A., and Hoogerbrugge, P. M. (1996b): Herpes Simplex Virus Thymidine Kinase gene therapy for rat malignant brain tumors. Hum. Gene Ther. 7, 197-205.
Weizsaecker, M., Deen, D. F., Rosenblum, M. L., Hoshino, T., Gutin, P. H., and Barker, M. (1981): The 9L brain tumor: description and application of an animal model. J. Neurol. 224, 183-192.
Wimperis, J. Z., Niemeyer, C. M., Sieff, C. A., Mathey-Prevot, B., Nathan, D. G., and Acreci, R. J. (1989): Granulocyte-macrophage colony-stimulating factor and interleukin-3 mRNA are produced by a small fraction of blood mononuclear cells. Blood 74, 1525.
Yokota, T., Lee, F., Rennick, D., Hall, C., Arai, N., Mosmann, T., Nabel, G., Cantor, H., and Arai, K. (1984): Isolation and characterization of a mouse cDNA clone that expresses mast-cell growth factor activity in monkey cells. Proc. Natl. Acad. Sci. USA 81, 1070.
Ziltener, H. J., Groth, B. F. d. S., Leslei, K. B., and Schrader, J. W. (1988): J. Biol. Chem. 263, 14511-14517. Zöller, M., Douvdevani, A., Segal, S., and Apte, R. N. (1992): Interleukin-1 produced by tumorigenic fibroblasts influences tumor rejection. Int. J. Cancer 50, 443-449.

**TABLE I**

| Overview of recombinant adenovirus vectors used in 'cracked cells' experiments | | | | |
|---|---|---|---|---|
| Recombinant adenovirus* | Therapeutic gene | E1 deletion | Promoter | Deposition number at ECACC |
| IG.Ad.CMV.rIL-3 | rat interleukin-3 | 459-3328 | Ad2 Major Late | V96071634 |
| IG.Ad.MLP.hIL-1α | human interleukin-1α | 459-3328 | CMV Immediate Early | |
| IG.Ad.CMV.TK | HSV - thymidine kinase | 459-3328 | CMV Immediate Early | |

| | | | | |
|---|---|---|---|---|
| *Construction of the vectors has been described in patent application EP 95 20 2213 | | | | |

## Claims

1. A composition for the treatment of malignancies comprising a relatively high burst dose of at least one cytokine or a functional equivalent or fragment thereof, a source for sustained release of a significantly lower but effective dose of at least one cytokine or a functional equivalent or fragment thereof together with a suitable vehicle for administration.

2. A composition according to claim 1 wherein the source of the lower dose of a cytokine is a gene delivery vehicle.

3. A composition according to claim 2 wherein the gene delivery vehicle is a recombinant vector.

4. A composition according to claim 3 wherein the vector is a viral vector.

5. A composition according to claim 4 wherein the viral vector is an adenoviral vector.

6. A composition according to anyone of the aforegoing claims wherein the source of the lower dose of a cytokine is a recombinant adenovirus.

7. A composition according to anyone of the aforegoing claims wherein at least one cytokine is interleukin-3 or interleukin-1 alpha.

8. A composition according to anyone of the aforegoing claims comprising an adjuvans.

9. A composition according to claim 8 wherein the adjuvans comprises inactivated cells.

10. A composition according to claim 9 wherein the inactivated cells are cracked cells.

11. A composition according to claim 9 or 10 wherein the cells are derived from recombinant adenovirus producing cells.

12. A composition according to claim 11 wherein the cells comprise a recombinant adenovirus according to claim 6.

13. A method for the treatment of malignancies comprising contacting said malignancies with a composition according to anyone of claims 1-12.

14. A method according to claim 13 for the treatment of solid tumors.

15. A method according to claim 14 further comprising inactivating treated cells from the solid tumor, mixing them with a suitable adjuvans and administrating said mixture to the mammal from which the tumor cells originate.

16. A vaccine for the treatment of solid tumors comprising inactivated autologous tumor cells, recombinant adenovirus, a cytokine or a functional equivalent or fragment thereof and an adjuvans.
